# EUROPEAN PATENT SPECIFICATION

(11) **EP 1 365 757 B1**
(45) Date of publication and mention of the grant of the patent: **28.12.2005**
(21) Application number: 02710627.7
(22) Date of filing: 05.02.2002
(51) Int. Cl.: A61K 31/138, A61P 27/16

(54) **Use of xylyloxy alkyl amines for treating tinnitus**
Verwendung von "xylyloxy alkyl amines" für die Behandlung von Tinnitus
Utilisation de "xylyloxy alkyl amines" pour le traitement de Tinnitus.

(30) Priority: 05.02.2001 SE 0100344
(43) Date of publication of application: 03.12.2003
(73) Proprietor: Isomex Limited, London EC2M 1JD (GB)
(72) Inventor: NORDMARK, Jan, S-113 57 Stockholm (SE)
(74) Representative: Fagerlin, Heléne
(86) International application number: PCT/SE2002/000196
(87) International publication number: WO 2002/062328

(56) References cited:
- DATABASE BIOSIS [Online] BIOSCIENCES INFORMATION SERVICE, PHILADELPHIA, PA, US; SAITO K ET AL : "The effect of mexiletine on tinnitus." Database accession no. prev198885051031 XP002902449 & PRACTICA OTOLOGICA KYOTO, vol. 80, no. 8, 1987, pages 1307 -1312,
- LENARZ T ET AL : "Tinnitus-therapie mit lidocain und tocainid." LARYNG. RHINOL. OTOL., vol. 64, 1985, pages 604-608, XP002902450

## Description

The present invention relates to the use of xylyloxy alkyl amines for preparing a pharmaceutical composition against tinnitus, a pharmaceutical composition comprising xylyloxy alkyl and aryl amines and a method for the treatment of tinnitus.

### Description

Tinnitus is a hearing disorder generally estimated to affect about 15% of the population in the US and Europe. In about 4% it is sufficiently severe to cause sufferers to seek medical help.

In a questionnaire investigation comprising 1091, 83% of patients with tinnitus were interested in obtaining treatment for their tinnitus (Lindberg P et al. Scand Audiol. 1984; 13:287-289). For about 1% of the population tinnitus is a disabling disease. Despite an immense effort and the evaluation of a number of agents from many trials, success in treating tinnitus has been very limited, and there is at present no well-established treatment providing long-term reduction of tinnitus impact in excess of placebo (Dobie, RA. Laryngoscope 1999;109:1202-1211). To improve the situation several recent reviews of the pharmacological treatment of tinnitus have emphasized the need for improved controlled trials, better measuring methods,and a better understanding of the underlying neurochemical mechanisms of the disease. As many studies of new drugs in this area have shown good effect initially, only to be shown later to be no more effective than placebo, uncontrolled studies cannot provide any idea of treatment-specific benefit (Dobie, op. cit.) Placebo effects are particularly strong in tinnitus. R A Dobie et al. (Am J Oto 1. 1993; 14:18-23) thought that probably the most important finding in their study on the effectiveness of the tricyclic antidepressant nortriptyline was that there were highly significant placebo effects in most of the variables used to measure the results. Had a placebo group not been included one might have concluded that tinnitus sensation was reduced by nortriptyline, but the difference in reduction was not significant (drug. 43%, placebo 30%).

In an unusually comprehensive double-blind cross-over study I Donaldson (J Laryngol Otol 1981; 95:947-951) asked 64 patients to evaluate the effect of lidocaine on their tinnitus and later the effect of the anticonvulsant carbamazepine. Four response groups were used to record degrees of success. The results confirmed previous reports on the efficiency for the short-term abolition of tinnitus in a large number of sufferers. Examination of the results for carbamazepine, however, failed to demonstrate any statistically significant difference between the positive results for the drug (45%) and placebo (35%). Two of the patients reported complete abolition of tinnitus from placebo.

The effectiveness of lidocaine (even if it cannot be used therapeutically due to its rapid elimination from the circulation) has led to attempts to get similar results for the closely related drugs tocainide and mexiletine (mex). Some studies have failed to find tocainide to be effective, but at least one investigation supports its effectiveness (Lenarz T, Gulzow J. Laryng Rhinol Otol. 1985; 64:604-608). A clinical cross-over study showed that tocainide infusion led to a 50% reduction in tinnitus intensity. Infusion with placebo,as in other placebo infusions,showed insignificant effect (5%). Tocainide is too toxic to be used therapeutically, but its pharmacological effectiveness seems assured.

There are only two investigations on mex reported in the literature. In 1981 MS McCormick and J S Thomas (Clin Otolaryngol. 1981; 6:255-258) gave 8 patients with unilateral tinnitus a bolus dose of 400 mg mex orally,and the patient's response was assessed by a matching technique at 5 different times during a 24 h. period. Based on the plasma levels at the time of the assessments the authors formed the hypothesis that tinnitus strength was reduced at plasma levels greater than 0.5 mg/l. In a double-blind cross-over trial 32 patients were then given 200 mg per day 3 times daily for 30 days. The hypothesis was not confirmed, however, and the drug was not superior to placebo.

In a later study (Saito, K et al. Practica Otologica Kyoto. 1987; 80: 1307-1312) 34 patients with tinnitus were given 300 mg mex per day orally for one or two weeks. There was no placebo group.Four response groups were used as in the Donaldson study described above, although with slightly less stringent criteria.In this form of representation the results were practically identical with the placebo results in the carbamazepine trial: 65% of the patients in both studies showed no effect, 14.6% of the subjects in the mex study showed an effect above 50% while 13 subjects in the placebo group in the carbamazepine study showed an effect above 60%. The authors of the mex study admit that 14.6% showing good effect is not a satisfactory result. They do suggest that the results showed that plasma levels above 0.7mg/l tended to show good relief, but this applies to only two patients, whereas three patients whose levels were above 0.7 mg/l showed less than 50% relief. There are methodological weaknesses in this study as well apart from the lack of placebo: the treatment period was very short, and there was no followup. The impression that this was a pilot study is strengthened by a comment in a later study (Saito, K et al. Practica Otologica Kyoto (Suppl.25).1988:15-20) where the authors write that as lidocaine and tocainide had showed effectiveness in tinnitus, they had expected that mex would be as well, but that their hopes were not fulfilled.

Neither of the two mex studies has been extended or replicated. The Saito et al. study has apparently never even been quoted by other researchers. Researchers in this field may not have found the results of either study promising enough to test mex again. The failure to show effect may depend on neglected factors. For a drug like mex with a half-life of about 8 hours, the difference between peak and trough after administration every 8 hours will be more than 30% (Zehender M et al. Zeitschr Kardiol. 1987;76:501-506). If the plasma level/response relationship is steep, there is a risk that the plasma level will not be therapeutically effective for part of the time.In this study on the antiarrhythmic effects of mex, the patients were given 200 mg 3 times daily - the same dosage regimen as in the McCormick and Thomas study on tinnitus. While the maximum plasma level was attained within 2 hours, at the end of the 8 hour dose interval it was subtherapeutic for almost 50 % of the patients.

There are more fundamental reasons why mex should have therapeutic value taking the molecular mechanims underlying tinnitus into consideration. J A Kaltenbach has recently presented a summary of new insights into the neural basis of tinnitus. The available evidence based on electrophysiological studies suggests that tinnitus is associated with disturbances in spontaneous neural activity in the auditory system. These abnormalities include increases in spontaneous activity (hyperactivity), changes in the timing of neural discharges, and an increase in bursting activity of neurons. Parallel studies using behavioural testing methods have demonstrated that agents which produce these neural changes, such as salicylate and quinine, also cause tinnitus in animals (J Am Acad Audiol. 2000; 11:125-137). Such studies have been used to localize an important center for tinnitus in the midbrain inferior colliculus. G D Chen and P J Jastreboff (Hear Res 1995; 82:158-178) found that salicylate induces an increase in spontaneous activity and the emergence of a bursting type of activity in the inferior colliculus which the authors proposed represented tinnitus-related activity. In another study Manabe, Y et al. (JORL Soc of Japan. 1998; 101:807-813) also found that salicylate given guinea pigs in doses giving a plasma concentration corresponding to that which induces tinnitus in humans also resulted in augmented spontaneous neural discharges. Repetitive neural activity is found in some diseases of the nerve and muscle. There is now a possible explanation for these abnormal events.

The normal transmission of electrical signalling in nerve and muscle requires a rapid opening and closing of voltage-activated Na (sodium) channels. Under certain conditions some channels fail to close leading to a small non-activating (or persistent, sustained, slow) Na current. The incomplete inactivation facilitates action potential generation, shortens interspike intervals and promotes burst discharges. In certain inherited muscle diseases, myotonias, and in a genetic cardiac condition called the Long QT syndrome the incomplete inactivation can be traced to specific modifications in the Na channels. In neural diseases no such mutations have yet been found, but the persistent Na currents which underlie the hyperexcitability of neural tissue in epilepsy and certain forms of pain are of a similar nature. In the last few years studies on these conditions have been greatly helped by the use of the sea anemone toxin ATXII. It differs from other toxins in selectively producing late, persistent sodium currents without affecting the peak current seen in normal nervous activity. It can thus alter the impulse behaviour in normal muscle and nerve to make it resemble that of diseased tissue. One effective therapy for both myotonia and certain forms of neuropathic pain syndromes is the use of low levels of intravenous levels of local anesthetics/anti-arrhythmics (e.g.lidocaine,mexiletine). These drugs have an action on Na channels opposite that of ATXII (Khodorova A. Muscle & Nerve. 2001; 24:634-647). Similarly in the cardiac LQT syndrome the mutations cause an increase in late currents leading to late openings with potentially serious consequences. Again lidocaine and mex are effective, reducing late current more than peak current. Also in epilepsy local application in the brain has been shown to cause the kind of neural bursts characteristic of the disease. Several authors have proposed that tinnitus is a form of sensory epilepsy, and others that it should be viewed as a form of chronic pain sensation. The last comparison according to a recent review is based on a number of similarities between the two conditions, and pain and tinnitus are often ameliorated by the same pharmacological agents, most notably certain tricyclic antidepressants, anticonvulsants such as phenytoin, and the local anesthetic lidocain. The similarities could indicate that both conditions are characterized by a similar lesion, and this has led to the evaluation of several pharmacological agents on the basis of their pain-relieving properties. Unfortunately, this approach has not yet led to an effective mode of treatment (Simpson J J, Davies WE.TIPS. 1999; 20:12-19).

It is not obvious why this model has failed. The local anesthetic effect of lidocaine depends on its Na channel blocking power. It has commonly been assumed that its effect on tinnitus has the same cause. The increased spontaneous activity in the inferior colliculus believed to be representative of tinnitus is abolished by lidocaine (Manabe et al, op cit). There is, however, no statistical correlation between Na channel blocking power and tinnitolytic effect. There are many compounds with greater blocking power than lidocaine which are without effect on tinnitus. Its pain-relieving properties in chronic pain are limited to particular neuropathic conditions.The plasma concentration in these cases is 10-20 times lower than that needed for blocking conduction in nerves. It has been suggested that the reason for this discrepancy might be that there are subgroups of nerves active in neuropathic pain particularly sensitive to lidocaine. A more relevant explanation in this connection would be that lidocaine is effective in counteracting the abnormal, repetitive impulse firing arising from incomplete inactivation in Na channels involved in several diseases of nerve and muscle and mimicked by application of ATXII to normal nerve and muscle fibers, as described by Khodorova et al. (op cit).

Similarly, although the effect of phenytoin on Na channels is the same as that of local anesthetics, the specific details of the blocking action if phenytoin seem to endow it with just the right properties to be a minimally toxic anticonvulsant, that is interfere with seizure-generating mechanisms in a highly selective fashion without the normal functioning of the brain being affected (Rogawski, MA. Advances in Neurol. 1998; 76:11-27).Numerous other drugs are much more potent Na channel blockers but are useless as anticonvulsants.

The mutation in the gene encoding the Na Channel causing the long QT syndrome for which ATXII serves as a model offers another example. Antiarrhythmics are divided into groups among which drugs of group 1 have effect on Na channels. Two members of group 1b, lidocaine and mex, are highly effective in the LTQ syndrome, whereas drugs in la are not, having an effect on cardiac muscle impulses similar to ATXII, not opposite as the 1b drugs, la group drugs have no relieving influence on tinnitus, quinidine in fact causing tinnitus, lb drugs, such as lidocaine, tocainide and phenytoin will ameliorate tinnitus. Among tricyclic antidepressants amitriptyline is the most effective in tinnitus. It has pain-relieving properties similar to lidocaine and phenytoin, and antiarrhythmic effects similar to local anesthetics.

Taking these facts into consideration a working hypothesis would be that only those antiarrhythmics/local anesthetics, anticonvulsants, and tricyclic antidepressants which have the same type of effect on Na channels and consequently an effect opposite that of ATXII are effective in tinnitus treatment. According to this hypothesis mex will be effective in tinnitus. There appears to be no exception to the rule. The only major group of drugs which have shown some effectiveness in tinnitus treatment is the benzodiazepines. In this case the effect is considered to depend on GABA stimulating action, as GABA is the major inhibitory transmitter in the nervous system. Interestingly, the drugs having the desired effect on Na channels, appear to have a stimulating effect on GABA as well. This has recently been shown for lidocaine, tocainide and mex (Nordmark J, Rydqvist B. Neuroreport.1997; 8:465-468, and unpublished observations). The effect on GABA may thus be contributing to the effect on tinnitus.

ATXII has not been used as a screening model for new drugs in pain or epilepsy treatment, but it has been used to develop derivatives of mex and tocainide in certain forms of myotonia. The model has been particularly valuable in the case of mex, which has a narrow therapeutic range, as it allows new drugs to be tested which can indicate greater effectiveness in reducing inactivating currents than in reducing normal currents (Desaphy JF et al. Neuromuscular Disorders. 1999; 9:182-189.Desaphy JF et al. Brit J Pharmacol.1999; 128: 1165-1174). Based on the results in myotonia studies and using the same ATXII model in tinnitus, the degree of effectiveness in reducing tinnitus intensity in increasing order will therefore be: (RS)-mex,(R-)-mex, (RS)1-(2,6,-dimethylphenoxy)-3-methyl-2-butanamine, (R-)-I-(2,6-dimethylphenoxy)-3-methyl-2-butanamine.

This does not necessarily indicate order of usefulness, as the side effect profile of the isopropyl derivative is not known.

### Summary of the invention

It has now surprisingly turned out that mexiletine is effective against tinnitus. Therefore, the invention relates to the use of substances with the formula I according to claim 1. i.e. mexiletine and its derivatives for the preparation of a pharmaceutical composition for treating tinnitus.

### Detailed description of the invention

In one aspect, the invention relates to the use of a compound having the formula I 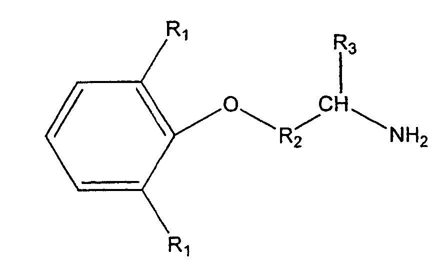 wherein
R1 are independently hydrogen or a C1-C4 straight or branched alkyl group, which may be substituted with one or more halogen groups and/or C1-C4 alkyloxy groups,
R2 is a C1-C3 hydrocarbyl group
R3 is a C1-C4 straight or branched alkyl group, which may be substituted by one or more C1-C4 alkyloxy groups and/or halogen groups
or R3 is a phenyl group, or a benzyl group, which may be substituted with one or more C1-C4 straight or branched alkyl group, and/or C1-C4 alkyloxy groups and/or halogen groups
or a pharmaceutically acceptable salt thereof for the preparation of a pharmaceutical composition for treating tinnitus.

Hydrocarbyl according to the invention refers to an organic radical composed primarily of carbon and hydrogen. The hydrocarbyl groups may be straight or branched chain alkyl, alkenyl or alkynyl groups which may, optionally be substituted with hydroxy or halogen groups. Typical hydrocarbyl groups of he present invention include straight and branched alkyl groups comprising 1, 2 and 3 carbon atoms such as methyl, ethyl, n-propyl. R2 is preferably a methylene or a ethylene group.

The above mentioned alkyl groups may be methyl, ethyl, n-propyl, s-propyl, n-butyl, s-butyl and t-butyl. The alkoxy groups alkyl-O- may comprise the same alkyl groups as mentioned for the alkyl groups as such.

The halogen groups may be chloride, fluoride or bromide.

Racemic compounds of mexiletine and analogues thereof are described in USP 3,954,872. The compounds may be produced by a stereospecific synthetic route which can be extended to include mexiletine analogues as well as mexiletine as described in the litterature (Carocci A et al. Chirality 2000;12:103-106). A method for the preparation of highly enantiopure stereoisomers of mexiletine has been described (Aav, R et al. Tetrahedron:Asymmetry 1999;10:3033-3038).

The invention especially relates to the use of a therapeutically effective compound comprising the racemate or the (R) enantiomer of a chiral compound having the fonnula I.

By comparing the potency for relieving allodynia from rat nerve root ligation by the mex enantiomers, which are known to stereoselectively inhibit sodium channels, CJ Sinnott et al (J Pain. 2000; 1:128-137) could show that pain relief was considerably greater for the (R-) enantiomer. (R-)-mex had several characteristics of previously reported relief by lidocaine, including a minimal threshold at a concentration of similar magnitude. (S+)-mex caused convulsive-like behaviour at a concentration, which had shown an absence of therapeutic activity, suggesting that toxicity might be caused by different mechanisms than those, which relieve allodynia. Wu W-P, Nordmark J et al. (Eur J Pain. 2000, 4:409-412) could also show a somewhat higher degree of toxicity for the (S+) isomer when injecting 25 mg/kg of the drug i.p.

In a different allodynia model 25 mg/kg of the mex i.v. injected enantiomers produced side effects in the case of the (S+) enantiomer so severe that pain measurements were not possible (Nordmark J and Schött E. Unpublished observations).

As will be shown in the example, the racemate of mexiletine has been shown to be effective in some patients in i.v. infusions of 500 mg during 2 hours. Given the greater potency of the (R-) isomers in pharmaceutical tests and in the relief of pain, there is reason to believe that the (R-) isomer will be more effective in the treatment of tinnitus than the racemate.

By performing tests as is shown in the example below it is possible to establish effective amounts of the (R-) isomers in the racemates. Thus, the practitioner can find optimal mixtures of the (R-) and (S+) isomers.

According to the invention a mixture of the isomers with a composition containing between 50% and 90% of the (R-) isomer may be used. Preferably the compound is substantially free of the (S+)-isomer and contains at least 90% by weight of the (R-) isomer and 10% or less of the (S+)-isomer. In the most preferred embodiment, the composition contains at least 98% by weight of the (R-) isomer. The substance is especially selected from mexiletine, i.e. 1-(2,6-dimethylphenoxy)-propanamine, the racemic or the (R-) enantiomer form of mexiletine or 1-(2,6-dimethylphenoxy)-3-methyl-2-butanamine and the racemic or the (R-) enantiomer form thereof. Another useful compound is α-[(2-methylphenoxy)methyl]-benzene-methaneamine.

Pharmaceutically acceptable acid addition salts of the compounds of formula I may be used according to the invention. They include but are not limited to hydrochloric acid, hydrobromic acid, fumaric acid, oxalic acid, malic acid, succinic acid, pamoic acid, sulphuric acid and phosphoric acid. Preferably the salt is a halide, carboxylate, sulfonate, or sulfate. Most preferably the salt is a chloride.

The type of tinnitus to be treated may be chronic, acute or subacute tinnitus.

The pharmaceutical compositions are prepared in a manner known to a person skilled in the pharmaceutical art. The carrier or the excipient could be a solid, semi-solid or liquid material that could serve as a vehicle or medium for the active ingredient. Suitable carriers or excipients are known in the art. The pharmaceutical composition could be adapted to oral, rectal, parenteral, or topical use such as a transdermal delivery patch and could be administered to the patient as tablets, capsules, solutions, suspensions or the like.

The pharmaceutical compositions could be administered orally, e.g. with an inert diluent or with an edible carrier, in the form of tablets, e.g. gelatine capsules, dragees and similar shaped or compressed pharmaceutical forms. For oral therapeutic administration the compounds according to the invention could be incorporated with excipients and used as tablets, lozenges, capsules, elixirs, suspensions, syrups, wafers, chewing gums and the like. These preparations should contain at least 4% by weight of the compounds according to the invention, the active ingredient, but could be varied according to the special form and could, suitably, be 4-70% by weight of the unit. The amount of the active ingredient that is contained in compositions is so high that a unit dosage form suitable for administration is obtained.

The tablets, pills, capsules, lozenges and the like could also contain at least one of the following adjuvants: binders such as microcrystalline cellulose, gum tragacanth or gelatine, excipients such as starch or lactose, disintegrating agents such as alginic acid, Primogel, corn starch, and the like, lubricants such as magnesium stearate or Sterotex, glidants such as colloidal silica dioxide, and sweetening agents such as saccharose or saccharin could be added or flavourings such as peppermint, methyl salicylate or orange flavouring. When the unit dosage form is a capsule it could contain in addition to the type above a liquid carrier such as polyethylene glycol or a fatty oil. Other unit dosage forms could contain other different materials that modify the physical form of the unit dosage form, e.g. as coatings. Accordingly, tablets or pills could be coated with sugar, shellac or other enteric coating agents. A syrup could in addition to the active ingredient contain saccharose as a sweetening agent and some preservatives, dyes and flavouring agents. Materials that are used for preparation of these different compositions should be pharmaceutically pure and non-toxic in the amounts used.

For parenteral administration the compounds according to the invention could be incorporated in a solution or suspension. Parenteral administration refers to the administration not through the alimentary canal but rather by injection through some other route, as subcutaneous, intramuscular, intraorbital, intracapsular, intrasternal, intravenous, intranasal, through eye drops or rectal (e.g. as a suppository, a cream or an ointment). These preparations could contain at least 0.1% by weight of an active compound according to the invention but could be varied to be approximately 0.1-50% thereof by weight. The amount of the active ingredient that is contained in such compositions is so high that a suitable dosage is obtained.

The solutions or suspensions could also comprise at least one of the following adjuvants: sterile diluents such as water for injection, saline, fixed oils, polyethylene glycols, glycerol, propylene glycol or other synthetic solvents, antibacterial agents such as benzyl alcohol or methyl paraben, antioxidants such as ascorbic acid or sodium bisulfite, chelating agents such as ethylene diamine tetraacetic acid, buffers such as acetates, citrates or phosphates, and agents for adjustment of the tonicity such as sodium chloride or dextrose. The parenteral preparation could be enclosed in ampoules, disposable syringes or multiple dosage vessels made of glass or plastic. Isotonic saline solutions containing 20-200 mg/ml may be used.
For topical administration the compounds according to the invention could be incorporated in a solution, suspension, ointment, or gel. These preparations could contain at least 0.1% by weight of an active compound according to the invention but could be varied to be approximately 0.1-50% thereof by weight. The amount of the active ingredient that is contained in such compositions is so high that a suitable dosage is obtained. Applying touch, pressure, massage, heat, warms, or infrared light on the skin, which leads to enhanced skin permeability, could facilitate the administration. Hirvonen, J., Kalia, YN, and Guy, RH. Transdermal delivery of peptides by iontophoresis, *Nat Biotechnol* 1996 Dec; 14(13): 1710-1713 describes how to enhance the transport of a drug via the skin using the driving force of an applied electric field. Preferably, iontophoresis is effected at a slightly basic pH.

As monitoring the mexiletine plasma level will be of little use in determining dosage due to the poor relationship found to exist between plasma level of the drug and pain relief (Galer B. Neurology 1995;45:S17-S25), or in the case of lidolcaine in tinnitus relief (Perucca E and Jackson P. J Laryngol Otol. 1985;99:657-661) the drug will have to be administered in increasing doses until the desired therapeutic effect or intolerable side effects are registered, as is routinely done in mexiletine therapy in neuropathic pain. For oral administration slow-release formulations are preferred as a means of keeping plasma drug concentration as constant as possible, thereby minimising minor side effects.

The dosage employed must still take age, weight and severity of the symptoms into consideration. Typically, the amount of mexiletine administered will be in the range of 400-1600 mg/day, or more preferably 600-1200 mg/day, but the actual decision as to dosage must be made by the attending physician. For the (R-) enantiomer the amount of drug administered will depend on the therapeutic effectiveness and on the side effects, but can be expected to be smaller.

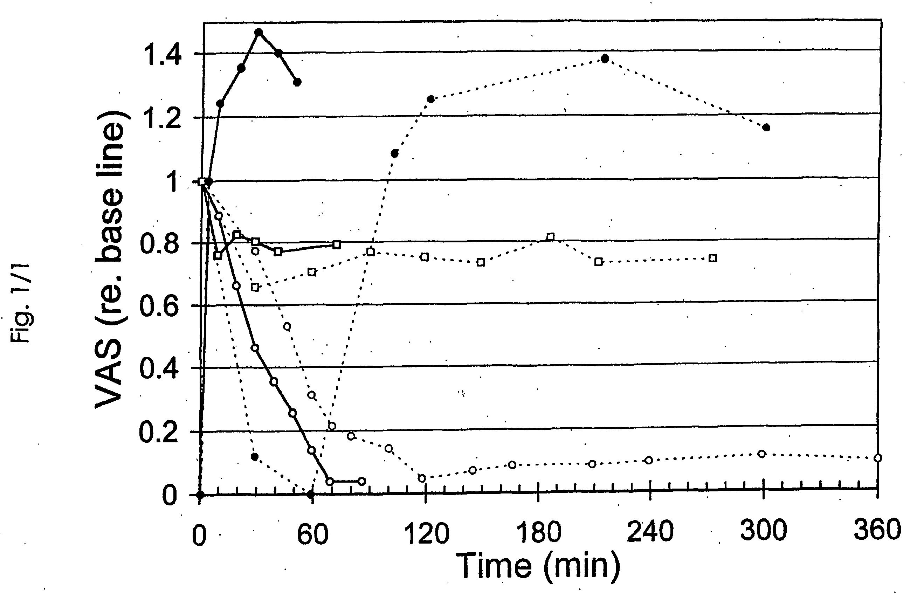

The invention will now be described with reference to the enclosed figure 1, which shows the visual analogue score with reference to tinnitus as a function of time after administration of lidocaine and mexiletine respectively to three different patients.
o refers to patient number 1
• refers to patient number 2 and
□ refers to patient number 3 in the examples.

Full lines indicate lidocaine administration and dotted lines mexiletine administration.

By the expression "comprising" we understand including but not limited to. Thus, other non-mentioned substances, additives or carriers may be present.

The invention will be illustrated by the following Example, which are only intended to illuminate and not restrict the invention in any way.

### Example 1

Patients at the Huddinge University Hospital Audiology clinic were evaluated for inclusion in the study on the effects of mexiletine on tinnitus. The patients fulfilling the inclusion criteria were given a clinical examination and a battery of audiological tests. Those willing to participate in the study were screened by giving 100mg lidocaine i.v. over 5 min. The intensity of the tinnitus was assessed by marking a VAS sheet every 5 min, where 12 indicated the worst tinnitus they could imagine and 0 indicated no tinnitus.

Patients experiencing reduced tinnitus were included in a further study where 500 mg mexiletine was slowly infused over 120 min. Tinnitus intensity was assessed every 10 or 20 min depending on the rapidity of change. At the same time blood samples were collected for later measurements of the mexiletine plasma levels.

Case 1, K.H., was a 40 year old woman with normal hearing suffering from left ear tinnitus. After lidocaine administration the tinnitus intensity was gradually reduced until 70 min after the start when it had been reduced by 96 %. The intensity reduction for mexiletine was similar, reaching 95 % after 118 min. Both curves remained at the 90-96% level until about 8 hours after the start of the test. There were no side effects.

Case 2, E.L., was a 46-year-old woman with severe hearing impairment above 3kHz who had suffered from bilateral tinnitus for 30 years. Less than one min after the start of the i.v. injection of lidocaine the tinnitus had vanished completely. There was no tinnitus 30s after injection. However, tinnitus returned to the initial level after about 2 min. It subsequently increased to a level of 46 % above the base level at 30 min, after which it slowly returned to this base level.

When 500 mg mexiletine were given tinnitus was reduced to a 60% level shortly before the infusion stopped. Tinnitus returned to normal after 1 hour. When given 550 mg over a period of 90 min, tinnitus had completely vanished after 60 min. It returned to base level after 100 min and increased in intensity by about 40% after 214 min, after which it gradually returned to normal. At the higher dose the patient experienced nausea.

Case 3, S.C., was a 44 year old man with normal hearing and bilateral tinnitus. Both lidocaine and mexiletine administration resulted in similar small reductions in intensity. There were no side effects.

As can be seen from the Figure, these cases show a similar pharmacodynamic profile for lidocaine and mexiletine. In case 1, both caused a strong reduction over an unusually long period. In case 2 the effects were dramatic but of short duration. In case 3 the reduction in tinnitus was moderate and of similar magnitude for both drugs. Correlation between plasma mexiletine level and tinnitus reduction was poor.

## Claims

1. Use of a compound having the formula I 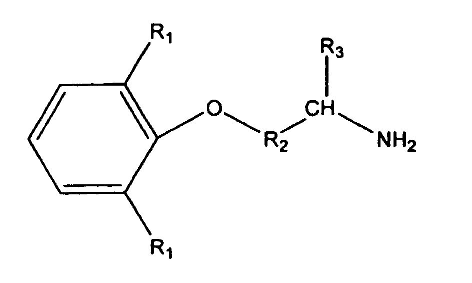 wherein
R1 are independently hydrogen or a C1-C4 straight or branched alkyl group, which may be substituted with one or more halogen groups and/or C1-C4 alkyloxy groups,
R2 is a C1-C3 hydrocarbyl group
R3 is a C1-C4 straight or branched alkyl group, which may be substituted by one or more C1-C4 alkyloxy groups and/or halogen groups or R3 is a phenyl group, or a benzyl group, which may be substituted with one or more C1-C4 straight or branched alkyl groups, and/or C1-C4 alkyloxy groups and/or halogen groups or a pharmaceutically acceptable salt thereof for the preparation of a pharmaceutical composition for treating tinnitus.

2. Use according to Claim 1, **characterised in that** R2 is a methylene or an ethylene group.

3. Use according to any of Claims 1-2, **characterised in that** the substance is the racemate of a chiral compound with the formula I.

4. Use according to any of Claims 1-2, **characterised in that** the substance is the (R-)-enantiomer of a chiral compound with the formula I.

5. Use according to Claim 1, **characterised in that** the substance is the racemate of mexiletine, i.e. of 1-(2,6-dimethylphenoxy)-propanamine.

6. Use according to Claim 1, **characterised in that** the substance is the (R-)-enantiomer of mexiletine, i.e. of 1-(2,6-dimethylphenoxy)-propanamine.

7. Use according to Claim 1, **characterised in that** the substance is selected from 1-(2,6-dimethylphenoxy)-3-methyl-butanamine, preferably in the racemic or the (R-) enantiomer form.

8. Use according to any of Claims 1-6, **characterised in that** the salt is a halide, carboxylate, sulfonate, or sulfate, preferably a chloride.

9. Use according to any of Claims 1-7, **characterised in that** the composition also comprises a pharmaceutically acceptable carrier or excipient.

## Patentansprüche

1. Verwendung einer Verbindung mit der Formel I 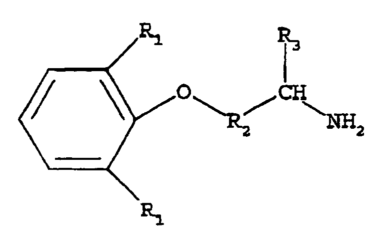 wobei
R₁ unabhängig Wasserstoff oder eine gerade oder verzweigte C1-C4-Alkylgruppe sind, die mit einer oder mehreren Halogengruppen und/oder C1-C4-Alkyloxygruppen substituiert sein können,
R2 eine C1-C3-Hydrocarbylgruppe ist,
R3 eine gerade oder verzweigte C1-C4-Alkylgruppe ist, die mit einer oder mehreren C1-C4-Alkyloxy-gruppen und/oder Halogengruppen substituiert sein kann, oder R3 eine Phenylgxuppe oder eine Benzylgruppe ist, die mit einer oder mehreren geraden oder verzweigten C1-C4-Alkylgxuppen und/oder C1-C4-Alkyloxygruppen und/oder Halogengruppen substituiert sein können,
oder eines pharmazeutisch akzeptablen Salzes davon zur Herstellung einer pharmazeutischen Zusammensetzung zur Behandlung von Tinnitus.

2. Verwendung nach Anspruch 1, **dadurch gekennzeichnet, dass** R2 eine Methylen- oder eine Ethylengruppe ist.

3. Verwendung nach einem der Ansprüche 1-2, **dadurch gekennzeichnet, dass** die Substanz das Racemat einer chiralen Verbindung mit der Formel I ist.

4. Verwendung nach einem der Ansprüche 1-2, **dadurch gekennzeichnet, dass** die Substanz das (R-)-Enantiomer einer chiralen Verbindung mit der Formel I ist.

5. Verwendung nach Anspruch 1, **dadurch gekennzeichnet, dass** die Substanz das Racemat von Mexiletin, d.h. von 1-(2,6-Dimethylphenoxy)propanamin, ist.

6. Verwendung nach Anspruch 1, **dadurch gekennzeichnet, dass** die Substanz das (R-)-Enantiomer von Mexiletin, d.h. von 1-(2,6-Dimethylphenoxy)propanamin, ist.

7. Verwendung nach Anspruch 1, **dadurch gekennzeichnet, dass** die Substanz ausgewählt ist aus 1-(2,6-Dimethylphenoxy)-3-methylbutanamin, vorzugsweise in der racemischen oder der (R-)-Enantiomeren-Form.

8. Verwendung nach einem der Ansprüche 1-6, **dadurch gekennzeichnet, dass** das Salz ein Halogenid, Carboxylat, Sulfonat oder Sulfat, vorzugsweise ein Chlorid, ist.

9. Verwendung nach einem der Ansprüche 1-7, **dadurch gekennzeichnet, dass** die Zusammensetzung auch einen pharmazeutisch akzeptablen Träger oder Hilfsstoff umfasst.

## Revendications

1. Utilisation d'un composé de formule 1 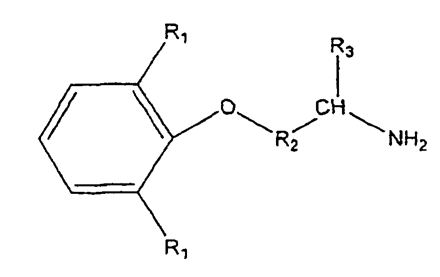 dans laquelle
R1 sont indépendamment un atome d'hydrogène ou un groupe alkyle à chaîne linéaire ou ramifiée en C1 à C4, qui peut être substitué avec un ou plusieurs groupes halogènes et/ou groupes alkyloxy en C1 à C4,
R2 est un groupe hydrocarbyle en C1 à C3
R3 est un groupe alkyle à chaîne linéaire ou ramifiée en C1 à C4, qui peut être substitué par un ou plusieurs groupes alkyloxy en C1 à C4 et/ou des groupes halogènes ou R3 est un groupe phényle, ou un groupe benzyle, qui peut être substitué avec un ou plusieurs groupes alkyle à chaîne linéaire ou ramifiée en C1 à C4, et/ou des groupes alkyloxy en C1 à C4 et/ou des groupes halogènes ou un sel acceptable d'un point de vue pharmaceutique de celui-ci pour la préparation d'une composition pharmaceutique pour traiter un acouphène.

2. Utilisation selon la revendication 1, **caractérisé en ce que** R2 est un groupe méthylène ou un groupe éthylène.

3. Utilisation selon l'une quelconque des revendications 1 à 2, **caractérisé en ce que** la substance est le racémate d'un composé chiral de formule 1.

4. Utilisation selon l'une quelconque des revendications 1 à 2, **caractérisé en ce que** la substance est l'énantiomère-(R-) d'un composé chiral de formule I.

5. Utilisation selon la revendication 1, **caractérisé en ce que** la substance est le racémate de mexilétine, c'est-à-dire de 1-(2,6-diméthylphénoxy)-propanamine.

6. Utilisation selon la revendication 1, **caractérisé en ce que** la substance est l'énantiomère -(R-) de mexilétine, c'est-à-dire de 1-(2,6-diméthylphénoxy)-propanamine.

7. Utilisation selon la revendication 1, **caractérisé en ce que** la substance est choisie parmi 1-(2,6-diméthylphénoxy)-3-méthyl-butanamine, de préférence sous la forme racémique ou énantiomérique (R-).

8. Utilisation selon l'une quelconque des revendications 1 à 6, **caractérisé en ce que** le sel est un halogénure, un carboxylate, sulfonate ou sulfate, de préférence un chlorure.

9. Utilisation selon l'une quelconque des revendications 1 à 7, **caractérisé en ce que** la composition comprend également un support ou excipient acceptable d'un point de vue pharmaceutique.
